# EUROPEAN PATENT APPLICATION

(11) **EP 4 749 626 A1**
(43) Date of publication of application: **27.05.2026**
(21) Application number: 24306977.0
(22) Date of filing: 26.11.2024
(51) Int. Cl.: G16B 15/30, G16C 10/00, G16C 20/50, G16C 20/70

(54) **MOLECULAR CONFIGURATION SAMPLING**

(71) Applicant: Université de Lorraine, 54000 Nancy (FR); Centre National de la Recherche Scientifique, 75016 Paris (FR); Wiczew, Daniel, 54500 Vandoeuvre-lès-Nancy (FR)
(72) Inventor: WICZEW, Daniel, 54500 Vand uvre-lès-Nancy (FR); TAREK, Mounir, 54410 Laneuveville-devant-Nancy (FR)
(74) Representative: Gevers & Orès

(57) **Abstract**

The invention relates to a method (100) for finding possible configurations of a molecule, comprising iteratively executing an episode comprising: - determining (102) configurations of the molecule from a starting configuration; - determining (106) a reward for each determined configuration, by, at each episode: optimizing (106-2) a target network (TN), from configurations already determined, to achieve a predefined goal, optimizing (106-4) the predictor network (PN), from configurations already determined, to try to replicate an output of the target network (TN), and for each configuration of the current episode, calculating (106-6) the reward from a comparison of the output of the optimized target network (TN) and an output of the optimized predicator network (PN); and - selecting (108) the configuration with the highest novelty reward as the starting configuration for the next episode.

## Description

### Technical field of the invention

The technical field of the invention described herein relates to computational molecular biology and bioinformatics, with a specific focus on methodologies for finding possible configurations of molecules including proteins, polynucleotide, such as ribonucleic acid (RNA) and deoxyribonucleic acid (DNA), or any other type of molecule. This invention uses advanced computational techniques and machine learning, including for example reinforcement learning and neural networks, to iteratively determine and evaluate novel molecular configurations without prior knowledge of the target structures. The process is designed to enhance the discovery of biologically or physically meaningful molecular configurations, which are crucial in fields like drug discovery. The technology addresses challenges in setting effective reward systems in configuration sampling and proposes a novel approach using unsupervised learning algorithms to guide the exploration and identification of new molecular configurations.

### Background of the invention

There are several known methods that help finding configurations of a given molecule, such as those seeding with experimental or Alphafold structures: FAST, Adaptive Bandit, Adaptive Sampling, REAP, TSMD, TALOS and Multiagent REAP, Metadynamics, umbrella sampling, classic and accelerated molecular dynamics, and many more. These methods are inspired by physics, machine learning or both.

However, defining a numerical signature (called a reward) of "how good and new" is a discovered molecular configuration in many scenarios proves challenging or impossible. Typically, a reward system, which measures the success of an effective sampling methods, relies on comparison to prior knowledge (using for example Root Mean Square Deviations or distances to a particular configuration derived from experiments, or from AlphaFold predictions). The latter serve as benchmarks in methods such as Adaptive Bandit, Adaptive Sampling, Steered Molecular Simulations, and Elastic Network-Driven Brownian Dynamics to guide the discovery process. Alternatively, by using specific molecular coordinate systems (collective variables or reaction coordinates) in techniques such as REAP, TSMD, TALOS, Multiagent REAP, and Metadynamics, one can explore configurations along predefined pathways. Discovery can also be achieved by biasing the physics of the system using external forces (accelerated molecular dynamics) which may result in inaccurate and non-physical data, or by employing very large computational resources (brute force classical molecular dynamics) where cost rises exponentially with size of the system. Without prior knowledge or availability of extremally large computational power, it becomes difficult, if not impossible to reveal significant novel molecular configuration. This hinders the automatic discovery of novel and potentially valuable molecular structures.

Besides, the article "Exploration by random network distillation", Y. Burda et al, 2018 describes calculating a numerical value (called "curiosity reward"), applied to frames of video games to avoid situation of exploring noisy areas of the game (called a "TV noise problem"). The value is higher for new, never-seen frames and lower for already seen video game frames.

It may thus be desired to provide a method for finding possible configurations of a molecule, which overcomes at least part of the aforementioned problems and constraints.

### Brief description of the invention

The invention relates to a method for finding possible configurations of a molecule, according to claim 1.

"configurations already determined" means configurations determined at the current episode and/or, at the episodes following the first episode, configurations determined at one or several of the previous episode(s).

In the invention, reward (called hereinafter molecule configuration reward, or simply mol. conf. reward) is defined so that it is possible to generally measure if molecular configurations are new useful configurations (physical, biological), without requiring prior knowledge of target configurations neither use of specific measures (collective variable, similarity to target experimental configuration). The mol. conf. reward is used as a criterion to select a molecular configuration that can be used as a starting point to explore other molecular configurations using more conventional frameworks (e.g. Molecular Dynamics).

The invention may also include one or more of the optional characteristics of claims 2 to 10, according to any technically possible combination.

The invention also relates to a computer program according to claim 11.

### Brief description of the drawings

The invention will be better understood with the help of the description which follows, given solely by way of example and made with reference to the appended drawings in which:
- figure 1 is a block diagram of a method according to the invention,
- figure 2 is a block diagram illustrating a first episode of an example implementation of the method of figure 1,
- figure 3 is a block diagram illustrating a second episode of the example implementation of the method of figure 1,
- figure 4 is a block diagram illustrating a third episode of the example implementation of the method of figure 1,
- figure 5 is a schematic representation of a neural network that can be used in the method of figure 1,
- figure 6 is a schematic representation of a computer system for carrying out the method of figure 1, and
- figure 7 illustrates the efficiency of the method according to the invention, with respect to other sampling methods.

### Detailed description of the invention

The invention relates to finding possible configurations of a molecule. Generally speaking, a molecule comprises several atoms, which may be divided into groups, each gathering one or several atoms, the groups being called residues. According to the present invention, the term "molecule" is generic and means either a single molecule or a portion of a molecule or a set of molecules. Furthermore, according to the present invention, a configuration of a molecule comprises at least spatial coordinates of at least some atoms and/or of at least some residues of the molecule.

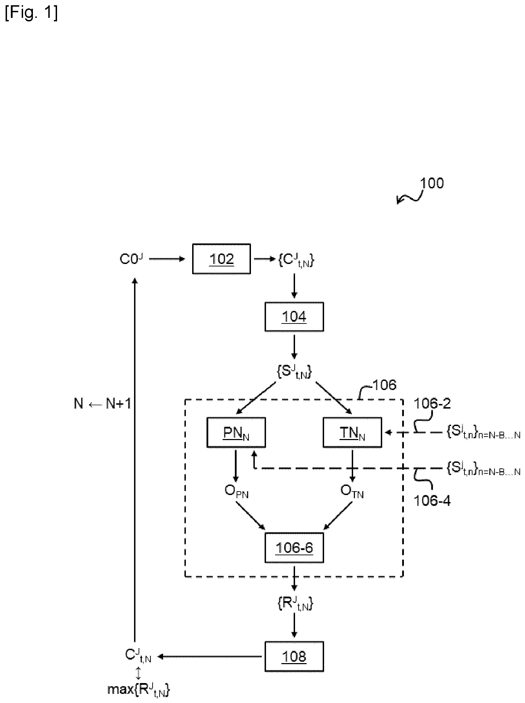

Referring to figure 1, a method 100 according to the invention for finding possible configurations of a molecule, will now be described.

The method 100 comprises iteratively executing a bloc of steps, called an episode, for at least one agent. In the following description, the index n will be used to identify each episode (n=1... N, with N ≥ 1 being the current episode) and the index j will be used to identify each agent (j=1...Nj, with Nj ≥ 1 the number of agents), the index J identifying the current agent. Each episode comprises the steps 102, 104, 106 below.

The method 100 first comprises a step 102 of determining configurations of the molecule from a starting configuration using a simulation with parameters, such as velocities of the atoms and/or residues of the molecule, randomly initialized for each episode. The determined configurations may be for example successive configurations in time. The simulation may be for example a molecular dynamics (MD) simulation. Each determined configuration is referenced C^{J}_{t,N}, where t is an index used to identify the configuration (t=1... Nt, with Nt ≥ 2 being the total number of configurations determined at each current episode N).

At the first episode (N=1), the starting configuration is referenced C0^{J}. The agents may start from the same starting configuration or different starting configurations.

As explained above, the simulation may be designed for simulating an evolution of the molecule at successive time steps, as it is for example the case for a molecular dynamics (MD) simulation. The configurations C^{J}_{t,N} may then be taken every Ns steps of the simulation. For example, Ns is equal to 1 000.

The simulation may for example use a predefined temperature, included for example between 270 K to 370 K, for example equal to 303 K, to calculate the output configurations from the starting configuration. The step 102 may then comprise applying a temperature boost (i.e. increasing the predefined temperature, for example of at least 50% when the temperatures are expressed in Kelvin) to steps of the simulation, for example the M first steps. For example, M is equal to 5 000. For example also, the increased temperature may be included between 400 K and 600 K, for example equal to 500 K.

The method 100 further comprises a step 104 of deriving features from each configuration C^{J}_{t,N}, the features being called a state, referenced S^{J}_{t,N}, of the configuration C^{J}_{t,N}. The features may comprise various measurable or computable aspects of a structure or properties of the molecule. For example, the features may comprise spatial attributes (for example, the spatial coordinates included in the associated configuration and/or derived spatial coordinates) and/or distance metrics (for example, distances between atoms or residues of the molecule and/or other relevant distances of the molecule, calculated from the spatial coordinates of the associated configuration) and/or angle-based measures (for example, dihedral angles between the atoms or the residues of the molecule and/or other relevant angular relationships influencing molecular structure and function, calculated from the spatial coordinates of the associated configuration).

The method 100 further comprises a step 106 of determining, for each successive configuration C^{J}_{t,N}, a reward, called molecule configuration reward (or simply mol. conf. reward) and referenced R^{J}_{t,N}. The mol. conf. reward R^{j}_{t,N} is designed to be higher as the configuration C^{j}_{t,N} is new compared to at least some of the configurations determined at the current episode N and, for the episodes following the first episode (N>1), the previous episode(s) 1... N-1, preferably for all agents. A configuration is not new and will be associated to a low mol. conf. reward, if similar configurations already appeared. On the contrary, a configuration is new and will be associated to a high mol. conf. reward, if no similar configuration has already appeared. For example, the mol. conf. reward R^{j}_{t,N} is determined from the state S^{j}_{t,N} corresponding to the configuration C^{j}_{t,N}.

Each mol. conf. reward R^{j}_{t,N} is determined by using a method based on Random Network Distillation (RND) (see Y. Burda et al., 2018), but modified to be adapted to the context of the invention, which will now be described.

The step 106 uses a target network TN, the same for all agents j, including parameters and designed to receive a state and to provide in response some output O_{TN}.

The parameters of the target network TN are optimized at each current episode N (instead of being fixed in the case of the RND of Burda et al., 2018), to provide an optimized target network, reference TN_{N}. To this end, at each current episode N, at a step 106-2, the target network TN_{N-1} is optimized from the states S^{j}_{t,n} (n=1... N), preferably from all agents j, for example of the current episode N and at least some of the previous episode(s) 1... N-1. For example, the states S^{j}_{t,n} of B previous episode(s) are used (B ≥ 1), so that the optimization uses the states S^{j}_{t,n}, with n=N-B... N. Using the states of all agents j allows them to share their experience, i.e. encountered molecular configurations. During the optimization, the parameters of the target network TN_{N-1} are modified to reach a certain goal, which can be any chosen goal. At the first episode (N=1), the initial target network, referenced TN₀, is for example initialized using any neural network initialization technique such as Lecun Normal.

Preferably, the target network TN is designed to carry out dimension reduction, i.e. to pass from a high-dimensional space of the input state into a low-dimensional space so that the low-dimensional representation retains only the most meaningful properties of the original data.

For example, the goal of the target network TN is to project the input configuration into a K-dimensional embedding, so that for example the output O_{TN} only changes if meaningful features (chemically, biologically or physically relevant features) of the molecular configuration change. In this case, the output O_{TN} may be provided by a projection stage of the target network TN and may comprise K embedding vectors respectively representing K slow dynamics processes. The target network TN is then optimized by a learning algorithm that ensures that the K embedding vectors represent the K slow dynamic processes. For example, the target network TN may be a modified VAMPnet (i.e. without the Softmax layer), for example as described in the article "VAMPnets for deep learning of molecular kinetics", Mardt et al., 2018, or in the article "State-Free Reversible VAMPNet", W. Chen et al., 2019. The VAMPnet requires a configuration at instant t and at an instant t plus a lag time. For this reason, the considered configuration C^{j}_{t,N} is for example provided to the modified VAMPnet along with the configuration C^{j}_{t+1,N} at time t+1 (generally, at time C^{j}_{t+L,N} at time t+L, L being a predefined lag time).

For example, when the target network TN is designed to output slow dynamics processes as explained above, the K outputs are merged to compute a variational score that is maximized to optimize the target network.

The step 106 further uses a predicator network PN, the same for all agents j, including parameters and designed, as well as the target network TN, to receive a state and to provide in response an output O_{PN}. At each current episode N (except the first one (N = 1)), at a step 106-4, the predicator network PN_{N-1} is optimized in order to try to replicate the output of the target network TN_{N}. The optimized predicator network at episode N is referenced PN_{N}. The predicator network PN_{N-1} is optimized from the states S^{j}_{t,n}, from all agents j, for example of the current episode N and of at least some of the previous episode(s) 1... N-1. For example, the optimization uses the states S^{j}_{t,n}, with n=N-B... N-1. For example, the optimization aims to minimizing the mean square error between the outputs: ∥O_{TN} - O_{PN}∥², using for example a gradient descent method. At the first episode (N=1), an initial predicator network PN₀ is used, initialized for example using any neural network initialization technique such as Lecun Normal.

At a step 106-6, each state S^{j}_{t,N} obtained at the current episode N is then provided to both the target network TN and the predicator network PN and theirs outputs O_{TN}, O_{PN} are compared to calculate the mol. conf. reward R^{j}_{t,N} for the state S^{j}_{t,N}. For example, the mol. conf. reward R^{j}_{t,N} is defined as the mean square error between the outputs O_{TN}, O_{PN}: R^{j}_{t,N} = ∥O_{TN} - O_{PN}∥². In this way, the predictor network PN is designed to give a good prediction of the output O_{TN} of the target network TN for a configuration similar to the previous configurations, so that the mol. conf. reward is low. In contrast, the output of the target network TN is difficult to predict by the predictor network PN for a configuration different from the previous configurations, so that the mol. conf. reward is high.

The method 100 further comprises a step 108 of selecting the configuration C^{j}_{t,N} with the highest mol. conf. reward R^{j}_{t,N} as the starting configuration for the next episode N+1.

The method 100 may be used to produce a new drug for a disease.

In this case, the method 100 is first carried out to find a new (stable) configuration of a protein which causes the disease.

Then, a drug able to bind to this configuration of the protein.

Then, the drug may be produced to be prescribed to patients with the disease.

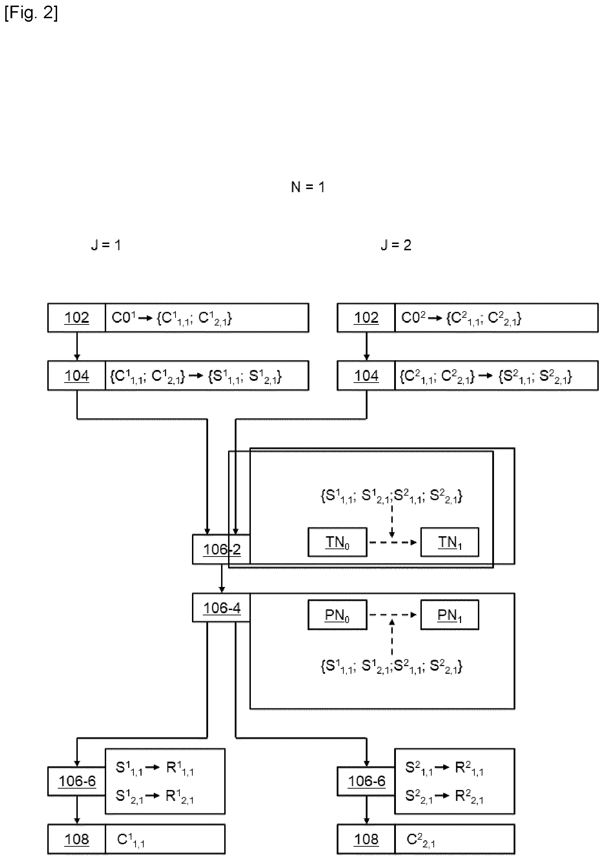

Referring to figure 2, figure 3 and figure 4, an example of carrying out the method 100 of figure 1 will now be described. In this simple example, there are only two agents (Nj=2), two configurations per episode (Nt=2) and the optimizations of the target network and the predictor network are based only on the current episode and the previous episode (B=1).

Referring to figure 2, the first episode (N=1) will now be described.

At the step 102 for the agent 1, configurations C¹_{1,1}, C¹_{2,1} are determined from an initial configuration C0¹.

At the step 104 for the agent 1, states S¹_{1,1}, S¹_{2,1} are respectfully determined from the configurations C¹_{1,1}, C¹_{2,1}.

Similarly, at the step 102 for agent 2, configurations C²_{1,1}, C²_{2,1} are determined from an initial configuration C0².

At the step 104 for the agent 2, states S²_{1,1}, S²_{2,1} are respectfully determined from the configurations C²_{1,1}, C²_{2,1}.

At the step 106-2, an initial target network TN₀, for example initialized using any neural network technique such as Lecun Normal, with random parameter, is optimized to give the optimized target network TN₁. The optimization uses the states determined for both agents at the current episode (there is no previous episode), i.e. the states S¹_{1,1}, S¹_{2,1}, S²_{1,1}, S²_{2,1}.

At the step 106-4, an initial predictor network PN₀, for example with random parameters, is optimized to give the optimized predictor network PN₁. The optimization uses the states determined for both agents at the current episode (there is no previous episode), i.e. the states S¹_{1,1}, S¹_{2,1}, S²_{1,1}, S²_{2,1}.

At the step 106-6 for the agent 1, the target network TN₁ and the predictor network PN₁ are used to determine mol. conf. rewards R¹_{1,1}, R¹_{2,1} of respectively the states S¹_{1,1}, S¹_{2,1}.

At the step 108 for the agent 1, the mol. conf. rewards R¹_{1,1}, R¹_{2,1} are compared to each other to find the highest and the corresponding configuration is selected, for example C¹_{1,1} in figure 2, to be used as starting configuration at the next episode of agent 1.

At the step 106-6 for the agent 2, the target network TN₁ and the predictor network PN₁ are used to determine mol. conf. rewards R²_{1,1}, R²_{2,1} of respectively the states S²_{1,1}, S²_{2,1}.

At the step 108 for the agent 2, the mol. conf. rewards R²_{1,1}, R²_{2,1} are compared to each other to find the highest and the corresponding configuration is selected, for example C²_{2,1} in figure 2, to be used as starting configuration at the next episode of agent 2.

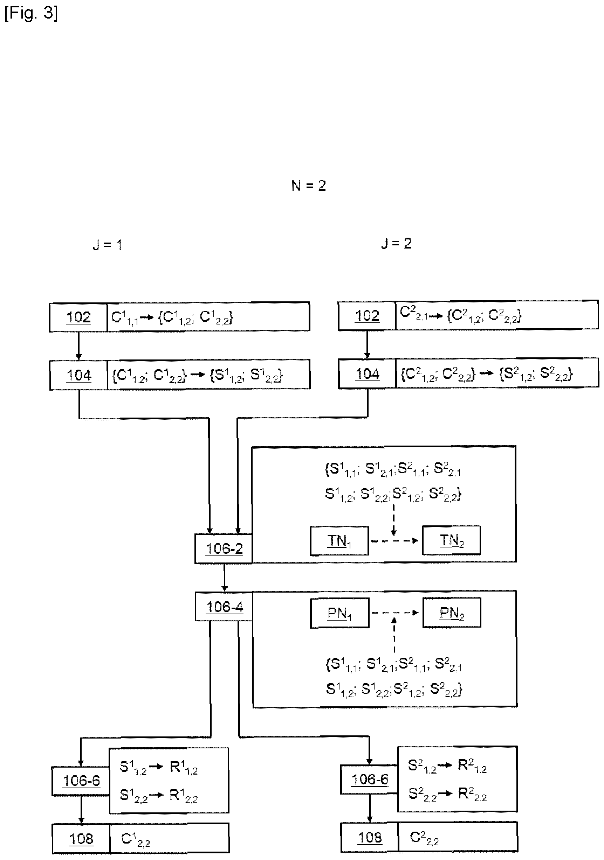

Referring to figure 3, the second episode (N=2) will now be described.

At the step 102 for the agent 1, configurations C¹_{1,2}, C¹_{2,2} are determined from the configuration C¹_{1,1} selected at the end of the episode 1, using the simulation with changed parameters, for example changed randomly. Because the parameters have changed with respect to the previous episode, the configuration C¹_{1,2} is different from the configuration C¹_{2,1}, although they both come from the configuration C¹_{1,1}.

At the step 104 for the agent 1, states S¹_{1,2}, S¹_{2,2} are respectfully determined from the configurations C¹_{1,2}, C¹_{2,2}, using the simulation with changed parameters, for example changed randomly. Because the parameters have changed with respect to the previous episode, the configuration C¹_{1,2} is different from the configuration C¹_{2,1}, although they both come from the configuration C¹_{1,1}.

Similarly, at the step 102 for agent 2, configurations C²_{1,2}, C²_{2,2} are determined from the configuration C²_{2,1} selected at the end of the episode 1.

At the step 104 for the agent 2, states S²_{1,2}, S²_{2,2} are respectfully determined from the configurations C²_{1,2}, C²_{2,2}.

At the step 106-2, the target network TN₁ is optimized to give the optimized target network TN₂. The optimization uses the states determined for both agents, i.e. the states S¹_{1,1}, S¹_{2,1}, S²_{1,1}, S²_{2,1}, S¹_{1,2}, S¹_{2,2}, S²_{1,2}, S²_{2,2}.

At the step 106-4, the predictor network PN₁ is optimized to give the optimized predictor network PN₂. The optimization uses the states of the previous episode, determined for both agents, i.e. the states S¹_{1,1}, S¹_{2,1}, S²_{1,1}, S²_{2,1}.

At the step 106-6 for the agent 1, the target network TN₂ and the predictor network PN₂ are used to determine mol. conf. rewards R¹_{1,2}, R¹_{2,2} of respectively the states S¹_{1,2}, S¹_{2,2}.

At the step 108 for the agent 1, the mol. conf. rewards R¹_{1,2}, R¹_{2,2} are compared to each other to find the highest and the corresponding configuration is selected, for example C¹_{2,2} in figure 2, to be used as starting configuration at the next episode of agent 1.

At the step 106-6 for the agent 2, the target network TN₂ and the predictor network PN₂ are used to determine mol. conf. rewards R²_{1,2}, R²_{2,2} of respectively the states S²_{1,2}, S²_{2,2}.

At the step 108 for the agent 2, the mol. conf. rewards R²_{1,2}, R²_{2,2} are compared to each other to find the highest and the corresponding configuration is selected, for example C²_{2,2} in figure 2, to be used as starting configuration at the next episode of agent 2.

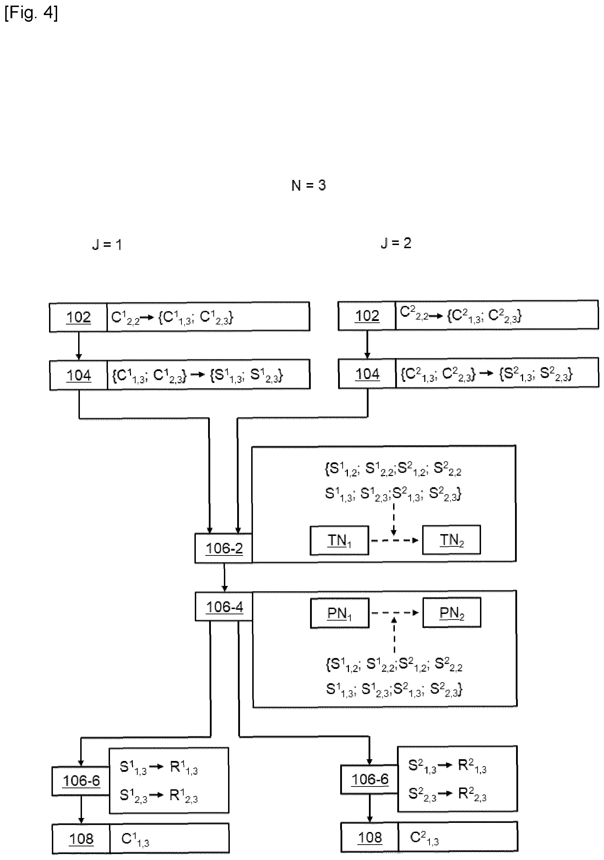

Referring to figure 4, the third episode (N=3) will now be described.

At the step 102 for the agent 1, configurations C¹_{1,3}, C¹_{2,3} are determined from the configuration C¹_{2,2} selected at the end of the episode 2.

At the step 104 for the agent 1, states S¹_{1,3}, S¹_{2,3} are respectfully determined from the configurations C¹_{1,3}, C¹_{2,3}.

Similarly, at the step 102 for agent 2, configurations C²_{1,3}, C²_{2,3} are determined from the configuration C²_{2,2} selected at the end of the episode 2.

At the step 104 for the agent 2, states S²_{1,3}, S²_{2,3} are respectfully determined from the configurations C²_{1,3}, C²_{2,3}.

At the step 106-2, the target network TN₂ is optimized to give the optimized target network TN₃. The optimization uses the states determined for both agents, i.e. the states S¹_{1,2}, S¹_{2,2}, S²_{1,2}, S²_{2,2}, S¹_{1,3}, S¹_{2,3}, S²_{1,3}, S²_{2,3}.

At the step 106-4, the predictor network PN₂ is optimized to give the optimized predictor network PN₃. The optimization uses the states of the previous episode, determined for both agents, i.e. the states S¹_{1,2}, S¹_{2,2}, S²_{1,2}, S²_{2,2}.

At the step 106-6 for the agent 1, the target network TN₃ and the predictor network PN₃ are used to determine mol. conf. rewards R¹_{1,3}, R¹_{2,3} of respectively the states S¹_{1,3}, S¹_{2,3}.

At the step 108 for the agent 1, the mol. conf. rewards R¹_{1,3}, R¹_{2,3} are compared to each other to find the highest and the corresponding configuration is selected, for example C¹_{2,3} in figure 2, to be used as starting configuration at the next episode of agent 1.

At the step 106-6 for the agent 2, the target network TN₃ and the predictor network PN₃ are used to determine mol. conf. rewards R²_{1,3}, R²_{2,3} of respectively the states S²_{1,3}, S²_{2,3}.

At the step 108 for the agent 2, the mol. conf. rewards R²_{1,3}, R²_{2,3} are compared to each other to find the highest and the corresponding configuration is selected, for example C²_{1,3} in figure 2, to be used as starting configuration at the next episode of agent 2.

Generally, any function approximator may be used as target network TN and predicator network PN, such as deep and shallow neural networks, quantum neural networks, spiking neural networks, linear approximations and linear transformations, tree based models, gradient boosting, bio-inspired neural networks, kernel based models, grid functions, linear and non-linear combination of several approximators.

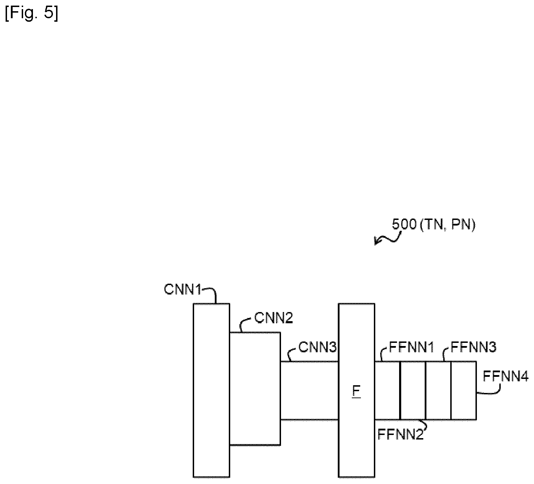

For example, referring to figure 5, an example 500 of a specific architecture of neural network that can be used as the target network TN and/or the predictor network PN will now be described. For example, the target network TN and the predictor network PN could have the same structure, i.e. for example the same sequence of layers of the same type. However, the layers of the two networks TN, PN may have different number of neurons or kernel size.

In the following, the network 500 will be described from input to output.

The network 500 first comprises several successive convolutional neural network (CNN) layers CNN1, CNN2, CNN3. For example, the layers CNN1, CNN2, CNN3 are each one dimensional CNN. In this case, the CNN layers may have successively increasing feature dimensions (represented in figure 5 with an increasing width) and decreasing sequence dimension (represented in figure 5 with a decreasing height).

The network 500 may further comprise a flatten layer F designed for projecting a tensor provided by the last CNN layer CNN3

The network 500 may further comprise several successive feed forward neural network layers, for example three hidden feed forward neural network layers FFNN1, FFNN2, FFNN3 and one output feed forward neural network layer FFNN4. Preferably, each of the FFNN layers of the predictor network PN comprises more neurons, for example at least ten times more neurons, than each of the FFNN layers of the target network TN.

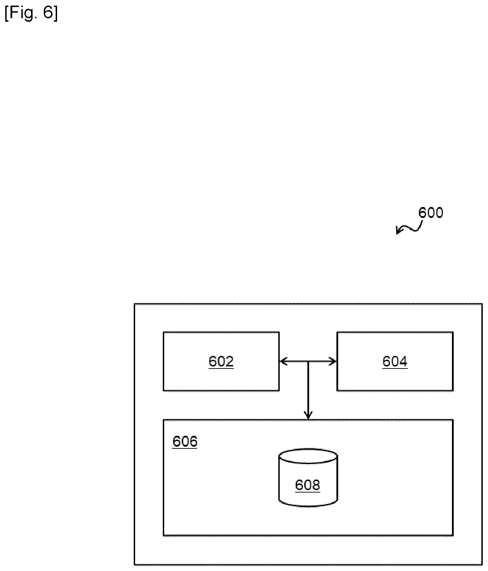

Referring to figure 6, the method 100 may be carried out on a computer system 600 comprising a data processing unit 602 (such as a microprocessor) and a main memory 604 (such as a "Random Access Memory" (RAM)) accessible by the processing unit 602. The computer system 600 further comprises for example a network interface and/or a computer-readable medium, such as for example a local medium (such as a local hard disk) or a remote medium (such as a remote hard disk accessible via the network interface through a communication network) or even a removable medium (such as a USB key, from the English "Universal Serial Bus", or a CD, from English "Compact Disc" or a DVD, from English "Digital Versatile Disc") readable using an appropriate drive of the computer system 600 (such as a USB port or a disc reader CD and/or DVD). A computer 608 program containing instructions for the processing unit 602 is recorded on the medium 606 and/or downloadable via the network interface. This computer program 608 is for example intended to be loaded into the main memory 604, so that the processing unit 602 executes its instructions, to carry out the steps of the method 100.

Alternatively, all or part of the steps of the method 100 could be implemented in the form by hardware modules, that is to say in the form of an electronic circuit, for example micro-wired, not involving a computer program.

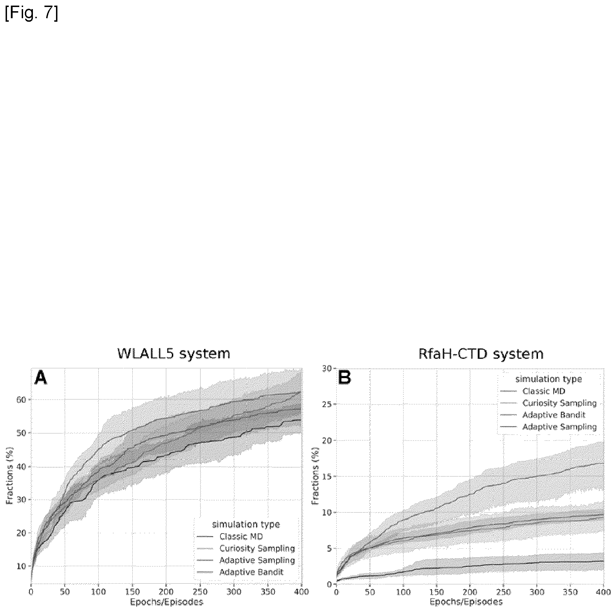

Referring to figure 7, the overall sampling performance of the method according to the invention has been compared to other methods, in the case of the WLALL5 peptide system (peptide of sequence Trp-Leu-Ala-Leu-Leu) (left plot) and the RfaH-CTDprotein system (Fold-180 Switching Protein RfaH) (right plot).

Each plot illustrates the percentage of sampled microstates (defined as clusters in the tICA space) compared to the ground truth data, as well as the confidence interval of 95% (shaded regions), for the classic MD method, the method of the invention, the adaptive sampling method (see Bowman et al., "Enhanced Modeling via Network Theory: Adaptive Sampling of Markov State Models", 2010), and the adaptive bandit method (see Pérez et al. « AdaptiveBandit: A Multi-armed Bandit Framework for Adaptive Sampling in Molecular Simulations », 2020).

In conclusion, it should be noted that the invention is not limited to the embodiments described above. It will indeed appear to those skilled in the art that various modifications can be made to the embodiments described above, in light of the teaching which has just been disclosed to them.

In the detailed presentation of the invention which is made previously, the terms used must not be interpreted as limiting the invention to the embodiments set out in the present description, but must be interpreted to include all the equivalents of which prediction is within the reach of those skilled in the art by applying their general knowledge to the implementation of the teaching which has just been disclosed to them.

## Claims

1. Method (100) for finding possible configurations of a molecule, comprising iteratively executing a bloc of steps, called an episode, each episode comprising:
- determining (102) configurations of the molecule from a starting configuration using a simulation;
- determining (106) a reward for each determined configuration, the reward being designed to be higher as the configuration is new compared to configurations already determined, by, at each episode:
• optimizing (106-2) a target network (TN), from configurations already determined, to achieve a predefined goal,
• optimizing (106-4) the predictor network (PN), from configurations already determined, to try to replicate an output of the target network (TN), and
• for each configuration of the current episode, calculating (106-6) the reward from a comparison of the output of the optimized target network (TN) and an output of the optimized predicator network (PN); and
- selecting (108) the configuration with the highest reward as the starting configuration for the next episode.

2. Method (100) according to claim 1, wherein, at each episode, the configurations determined from the starting configuration, are successive configurations in time and wherein parameters of the simulation are changed at each episode, for instance randomly.

3. Method (100) according to claim 1 or 2, wherein the simulation is a molecular dynamics simulation or a Monte Carlo simulation.

4. Method (100) according to any of claims 1 to 3, wherein the target network (TN) is designed to carry out dimension reduction.

5. Method (100) according to any of claims 1 to 4, wherein the target network (TN) is optimized (106-2) to maximize a score computed from the output of the target network (TN).

6. Method (100) according to any of claims 1 to 5, wherein the target network (TN) is designed to project the input configuration into K stable configurations, also called slow processes.

7. Method (100) according to claim 6, wherein the target network (TN) comprises a projection stage providing the output of the target network (TN), the output of the target network (TN) comprising K-dimensionnal real valued vectors respectively associated with the K slow dynamics processes.

8. Method (100) according to any of claims 1 to 7, wherein each episode is executed for several agents, and wherein the target network (TN) and the predictor network (PN) are shared between the agents.

9. Method (100) according to claim 8, wherein the target network (TN) is optimized (106-2) from configurations already determined, of at least some of the agents, preferably at least two of the agents, for example all of the agents.

10. Method (100) according to claim 7 or 8, wherein the predictor network (PN) is optimized (106-4) from configurations already determined, of at least some of the agents, preferably at least two of the agents, for example all of the agents.

11. Computer program (608) downloadable from a communications network and/or recorded on a computer-readable medium, **characterized in that** it includes instructions for executing the steps of a method (100) according to any one of claims 1 to 10, when said program is executed on a computer (600).
